# EUROPÄISCHE PATENTANMELDUNG

(11) **EP 1 063 225 A2**
(43) Veröffentlichungstag der Anmeldung: **27.12.2000**
(21) Anmeldenummer: 00113365.1
(22) Anmeldetag: 23.06.2000
(51) Int. Cl.: C07C 69/54, C07C 67/08, C07C 67/62

(54) **Verfahren zur Herstellung von (Meth)acrylsäureestern**

(30) Priorität: 25.06.1999 DE 19929258
(71) Anmelder: BASF AKTIENGESELLSCHAFT, 67056 Ludwigshafen (DE)
(72) Erfinder: Paulus, Wolfgang, 55270 Ober-Olm (DE); Reich, Wolfgang, 67133 Maxdorf (DE); Beck, Erich, 68526 Ladenburg (DE); Jaworek, Thomas, 67169 Kallstadt (DE); Königer, Rainer, 67251 Freinsheim (DE)
(74) Vertreter: Kinzebach, Werner, Dr.

(57) **Zusammenfassung**

Verbindungen mit alkoholischen Hydroxylgruppen werden in Gegenwart von Kupfer (II) salzen oder Gemischen davon als Polymerisationsinhibitoren mit Acryl- oder Methacrylsäure in Gegenwart eines Kohlenwasserstoffs als Wasserschleppmittel unter Auskreisen des Reaktionswassers bei 90 bis 150 °C verestert. Nach der Veresterung wird das Kupfersalz als Kupfersulfid gefällt und vom Ester abgetrennt. Die in hohen Ausbeuten resultierenden hellfarbigen (Meth) acrylester sind besonders für farbig pigmentierte Beschichtungen auf Substraten wie Holz, Papier, mineralischen Baustoffen, Kunststoff oder Metall geeignet.

## Beschreibung

Die Erfindung betrifft ein verbessertes Verfahren zur Herstellung heller Ester der Acrylsäure und/oder Methacrylsäure von ggf. alkoxylierten Alkoholen in Gegenwart eines sauren Veresterungskatalysators und einer Kupferverbindung als Polymerisationsinhibitor.

Strahlungshärtbare Bindemittel auf Basis von monomeren und oligomeren Acrylsäureestern von Alkoholen haben durch ihre lösungsmittelfreie und leichte Verarbeitbarkeit ein zunehmendes Marktinteresse als Lackharze und andere Beschichtungsmittel gefunden. Daher besteht an verbesserten Herstellungen der (Meth)acrylate von insbesondere mehrwertigen Alkoholen, ihren alkoxylierten Derivaten, Polyetherpolyolen oder Polyesterpolyolen ein anhaltender Bedarf, wie die zahlreichen Patentanmeldungen auf diesem Gebiet belegen. So sei als Stand der Technik auf die deutschen Patentanmeldungen DE-A 3316593, DE-A 3704098, DE-A 3843854, DE-A 3843930, DE-A 3843 938, DE-A 4019788 und DE-A 4430086 verwiesen.

Die Veresterung von Alkoholen und insbesondere Polyolen mit Methacrylsäure und bevorzugt Acrylsäure ist vielfach beschrieben. Außer einer Herstellung durch Umesterung ist der bevorzugte Weg der Herstellung von (Meth) acrylaten die Direktveresterung von Alkoholen bzw. Polyolen mit Acrylsäure oder Methacrylsäure in Gegenwart eines Veresterungskatalysators und eines Lösungsmittels, das mit Wasser ein azeotropes Gemisch bildet und als Wasserschleppmittel dient. Zur Beschleunigung der Veresterungsreaktion ist die Wahl von Reaktionstemperaturen von über 90 und insbesondere über 100 °C erwünscht, ebenfalls zur nachfolgenden raschen destillativen Entfernung der meist im Überschuß angewendeten Acrylsäure und/oder Methacrylsäure. Hohe Reaktionstemperaturen erfordern zur Erzielung guter Ausbeuten an den Veresterungsprodukten und zur wirksamen Unterdrückung der Polymerisation von (Meth) acrylsäure und deren Estern die Anwendung höherer Mengen von Polymerisationsinhibitoren. Da diese auch nach der Abdestillation überschüssiger (Meth) acrylsäure im Reaktionsprodukt verbleiben, ist zur Herstellung von hellen Veresterungsprodukten die Mitverwendung stark färbender Polymerisationsinhibitoren wie von Kupfersalzen, Phenothiazin, Hydrochinon und deren Derivaten nicht zweckmässig. Sie führen im allgemeinen zu dunklen Endprodukten. Die an sich bekannte Verwendung eines Kupfersalzes als Polymerisationsinhibitor bei der Herstellung von (Meth) acrylsäureestern erlaubt zwar hohe Veresterungstemperaturen, zur Herstellung heller Produkte mußte das Kupfersalz dann in einem Waschschritt ausgewaschen werden, was zeitlich aufwendig ist, die Esterausbeute senkt, den Bedarf an wieder abzudestillierenden Lösungsmitteln als Wasserschleppmittel erhöht und zudem zu einer hohen Abwasserbelastung führt.

Der vorliegenden Erfindung lag daher die Aufgabe zugrunde, ein einfaches Verfahren zur Herstellung von (Meth) acrylsäureestern zur Verfügung zu stellen, das helle Veresterungsprodukte in guter Ausbeute ergibt.

Es wurde nun gefunden, daß die gewünschten Ziele erreicht werden können mit einem Verfahren zur Herstellung von (Meth) acrylsäureestern durch Veresterung von Acrylsäure und/oder Methacrylsäure mit mindestens einer Verbindung mit einer oder mehreren alkoholischen Hydroxylgruppen bei einer Veresterungstemperatur von 90 bis 150 °C in Gegenwart eines sauren Veresterungskatalysators und eines Polymerisationsinhibitors unter Zugabe eines Kohlenwasserstoffs mit einem Siedepunkt im Bereich von 40 bis 120 °C als Wasserschleppmittel, wobei nach der Veresterung das Wasserschleppmittel und überschüssige (Meth) acrylsäure bei einer Temperatur von 90 bis 150 °C bei Normaldruck oder Unterdruck abdestilliert wird und gegebenenfalls das nach dem Abdestillieren erhaltene saure Produkt mit mindestens einer basischen anorganischen Verbindung neutralisiert wird, das dadurch gekennzeichnet ist, daß als Polymerisationsinhibitor ein Kupfer (II) salz, ein Kupfer (I)-salz oder ein Gemisch davon verwendet wird, wobei das Kupfer nach der Veresterung als Kupfersulfid gefällt und abgetrennt wird.

Das erfindungsgemäße Verfahren verbindet somit die Vorteile der Anwendung einer hohen Temperatur für eine schnellere Veresterung und rasche destillative Entfernung der überschüssigen (Meth) acrylsäure mit dem Vorteil eines niedrigen Bedarfs an Lösungsmittel als Wasserschleppmittel, dem Vorteil einer wirksamen Entfernung des Kupfersalzes als Polymerisationsinhibitor ohne Anwendung eines Waschschrittes mit dem Vorteil der Herstellung heller Endprodukte (Jodzahl ≤ 5, vorzugsweise ≤ 3, insbesondere ≤ 2) in guter Ausbeute.

Als Säuren für die Veresterungsreaktion werden Methacrylsäure, Acrylsäure oder Gemische dieser Säuren verwendet, wobei als Säure die Acrylsäure bevorzugt ist.

Als Verbindungen mit einer oder mehreren alkoholische Hydroxylgruppen kommen neben Alkoholen wie Laurylalkohol oder 2-Ethylhexylalkohol insbesondere Polyole mit 2 bis 6 alkoholischen Hydroxylgruppen in Frage. Beispiele von Verbindungen mit alkoholische Hydroxylgruppen sind Alkohole mit 2 bis 20, bevorzugt 2 bis 10 C-Atomen wie Ethylenglykol, Propylenglykol, 1,2-, 1,3- und 1,4-Butandiol, Neopentylglykol, Di- und Triethylenglykol, Di-und Tripropylenglykol, 1, 5-Pentandiol, 1, 6-Hexandiol, Glycerin, Trimethylolethan, Trimethylolpropan, Ditrimethylolpropan, Sorbit, Pentaerythrit oder Di-pentaerythrit. Sehr geeignet als Komponente mit alkoholischen Hydroxylgruppen sind auch Alkoxylierungsprodukte solcher Alkohole und insbesondere ethoxylierte und/oder propoxylierte mehrwertige Alkohole wie oxethyliertes Trimethylolpropan, ethoxyliertes und/oder propoxyliertes Pentaerythrit. Allgemein enthalten solche alkoxylierten Alkohole 1 bis 20 und bevorzugt 1 bis 10 Alkoxygruppen im Polyol-Molekül. Als Polyole können neben Polyetherpolyolen auch Polyesterpolyole, ethermodifizierte Polyesterpolyole, mehrere aliphatische Hydroxylgruppen aufweisende Polyepoxidharze oder entsprechende Polyurethane verwendet werden. Besonders geeignet für die Veresterungsreaktion sind dabei flüssige bis viskose niedermolekulare und oligomere Verbindungen mit alkoholischen Hydroxylgruppen.

Für die Veresterungsreaktion werden die Säure- und Hydroxylgruppen enthaltenden Komponenten insbesondere in Mengen von 1 bis 1,5 Mol Säure pro Hydroxylgruppe der Mono- oder Polyolkomponente eingesetzt. Jedoch kann die Säuremenge auch entsprechend herabgesetzt sein, wenn nur ein Teil der Hydroxylgruppen von Polyolen in Acrylate und/oder Methacrylate überführt werden soll.

Als saure Veresterungskatalysatoren kommen starke organische oder anorganische Säuren in Frage, die allgemein in Mengen von 0,1 bis 5 Gew.%, bezogen auf die Summe der Mengen von Alkohol- und (Meth) acrylsäurekomponente, eingesetzt werden. Bevorzugte saure Veresterungskatalysatoren sind Schwefelsäure, p-Toluolsulfonsäure, Methansulfonsäure sowie stark saure Ionenaustauscher.

Die Veresterung kann bei Normaldruck, Überdruck oder Unterdruck erfolgen.

Als Polymerisationsinhibitoren werden bei dem erfindungsgemäßen Verfahren für die Veresterungsreaktion und das Abdestillieren der überschüssigen (Meth)acrylsäure Kupfer (II) salze, insbesondere Kupfer (II) chlorid, Kupfer (II) nitrat oder Kupfer (II) sulfat, oder ein Gemisch aus einem Kupfer (II) salz und einem Kupfer (I) salz verwendet. Die Menge an Inhibitoren soll dabei für eine wirksame Unterdrückung der Polymerisation der (Meth) acrylsäure und deren Ester während der Veresterungsreaktion und dem nachfolgenden Abdestillieren der überschüssigen (Meth) acrylsäure hinreichend sein. Bevorzugt ist, die Kupfersalze in einer Menge von mindestens 0,02 und insbesondere von 0,2 bis 0,6 Gew.%, bezogen auf die resultierende (Meth) acrylsäureestermenge, zu verwenden.

Es hat sich als vorteilhaft erwiesen, dem Veresterungsgemisch vor der Veresterungsreaktion zusätzlich als Farbstabilisator unterphosphorige Säure (H₃PO₂), Triphenylphosphit oder eine Organophosphonsäure wie 1-Hydroxyethan-1,1-di-phosphonsäure in einer Menge von 0,01 bis 3 Gew.%, bezogen auf die resultierende Estermenge, zuzusetzen. Weitere übliche Polymerisationsinhibitoren, Farbstabilisatoren etc. wie Hydrochinonverbindungen, z.B. Hydrochinonmonomethylether, können im erfindungsgemäßen Verfahren nach dem Abdestillieren der überschüssigen (Meth) acrylsäure dem Ansatz in üblichen Mengen zugesetzt werden, ohne die Qualität der Verfahrensprodukte wesentlich zu beeinträchtigen.

Die Veresterung der Verbindung(en) mit alkoholischen Hydroxylgruppen bzw. Polyole mit (Meth)acrylsäure wird bei einer Temperatur von 90 bis 150 °C, insbesondere 95 bis 140 °C, bevorzugt 100 bis 130 °C und besonders bevorzugt bei 110 bis 120 °C, unter Rühren bei Normal-, Über- oder Unterdruck in Gegenwart eines geeigneten Kohlenwasserstoffs als Wasserschleppmittel zur Entfernung des bei der Veresterung anfallenden Reaktionswassers durchgeführt. Geeignete Kohlenwasserstoffe sind aliphatische, cycloaliphatische oder aromatische Kohlenwasserstoffe, die mit dem Reaktionswasser ein Azeotrop bilden. Sie sollen bevorzugt einen Siedepunkt oder Siedepunktsbereich zwischen 60 und 150 °C und bevorzugt zwischen 70 bis 140 °C aufweisen. Beispiele geeigneter Kohlenwasserstoffe als Wasserschleppmittel sind n-Heptan, Cyclohexan, Methylcyclohexan, Benzol, Toluol, Xylol-Isomere, Spezialbenzine und handelsübliche Kohlenwasserstoffgemische mit den angegebenen Siedepunkten oder Siedebereichen. Es ist ein Vorteil des erfindungsgemäßen Verfahrens, daß nur relativ geringe Mengen von Wasserschleppmittel erforderlich sind. Im allgemeinen werden sie in einer Menge von 0 bis 35, insbesondere in einer Menge von 1 bis 20 und bevorzugt in einer Menge von 2 bis 10 Gew.%, bezogen auf das Gemisch von (Meth) acrylsäure und Hydroxylverbindung, verwendet. Während der Veresterung wird das entstehende Reaktionswasser ausgekreist, wodurch auch das Ende der Veresterungsreaktion erkennbar ist.

Für die Veresterungsreaktion und für das Abdestillieren der überschüssigen (Meth) acrylsäure hat sich als vorteilhaft erwiesen, den Reaktor mit einem Inertgas-Luft-Gemisch mit einem Sauerstoffgehalt von 1 bis 20 Volumenprozent (Magerluft) zu durchspülen oder feinverteilt zu durchperlen, wobei der Gasstrom bevorzugt perlend durch das Reaktionsgemisch und/oder oberhalb vom Reaktionsgemisch durch den Reaktor geleitet wird. Der Gasstrom von Magerluft kann dabei dazu benutzt werden, das bei der Veresterung entstehende Reaktionswasser aus dem Reaktor zu schleusen. Dabei wird der Gasstrom während der Veresterung so begrenzt, daß er neben dem Reaktionswasser möglichst wenig an (Meth) acrylsäure, Hydroxylverbindung oder (Meth) acrylester aus dem Reaktor entfernt.

Nach beendeter Veresterung, bei der ein Veresterungsgrad von mindestens 85 und insbesondere 90 bis 95 % erzielt werden soll, wird der Kohlenwasserstoff und dann die überschüssige (Meth) acrylsäure bei einer Temperatur von 90 bis 150 °C, insbesondere bei 95 bis 140 °C, bevorzugt bei 100 bis 130 °C und besonders bevorzugt bei 110 bis 120 °C abdestilliert, wobei vorteilhaft bei Unterdruck, insbesondere bei einem Druck von 10 bis 150 mbar und bevorzugt bei 30 bis 80 mbar, gearbeitet wird und zweckmäßig Magerluft bei der Einstellung des Unterdrucks verwendet wird.

Das nach der Abdestillation resultierende Reaktionsprodukt ist sauer, da es den sauren Veresterungskatalysator und gegebenenfalls noch kleine Mengen (Meth) acrylsäure enthält. Vorzugsweise wird das Produkt daher neutralisiert. Dies erfolgt zweckmäßigerweise nach Abkühlen auf ca. 20 bis 100 °C. Die Neutralisation kann unter Naßbedingungen unter Verwendung von wässrigen basischen Lösungen wie von Natronlauge, Kalilauge oder wässrigen Sodalösungen erfolgen. Das saure Reaktionsrohprodukt kann aber auch einer sogenannten trocknen Neutralisation unterworfen werden, d.h. ihm werden im (Meth) acrylester nicht lösliche trockene Neutralisationsmittel zugegeben, wie Hydroxide, Oxide oder Carbonate von Alkalimetallen, Erdalkalimetallen und/oder von Aluminium. Entstehendes Neutralisationswasser kann danach z.B. durch Anlegen eines Vakuums entfernt werden.

Gemäß dem erfindungsgemäßen Verfahren wird das noch als Inhibitor enthaltene Kupfersalz als Kupfersulfid gefällt und abgetrennt. Unter dem Ausdruck "nach der Veresterung" ist jeder Zeitpunkt im Zuge der weiteren Aufarbeitung nach beendeter Umsetzung zu verstehen. Die Fällung kann insbesondere nach dem Abdestillieren der überschüssigen (Meth) acrylsäure oder vorzugsweise nach vollständiger oder teilweiser Neutralisation des sauren Reaktionsproduktes erfolgen. Die Fällung kann auch gleichzeitig mit der Neutralisation vorgenommen werden.

Die Fällung erfolgt durch Zugabe eines Alkalimetall- oder Ammoniumsulfids oder eines Alkalimetall- oder Ammoniumhydrogensulfids. Das Sulfid kann in fester Form, z. B. als Pulver oder als möglichst hochkonzentrierte wäßrige oder wäßrig-alkoholische Lösung zugegeben werden. Das gebildete Kupfersulfid wird dann im Zuge der nachfolgenden Verfahrensschritte, z. B. vor der Neutralisation, nach der Teilneutralisation oder vorzugsweise nach vollständiger Neutralisation, in üblicher Weise entfernt, z. B. durch Filtrieren mit Hilfe eines Druckfilters. Versuche, das Kupfersalz durch starke Komplexbildner wie mit Trilon®-Marken (Ethylendiamintetraessigsäure) oder Polyvinylpyrrolidon abzutrennen, führten nicht zum Erfolg. Es verblieb ein zu hoher Kupfergehalt, der bei zugabe von Amin zu einer Blau- oder Grünfärbung des Produkts führte. Das nachstehende Beispiel 1 zeigt, daß dies auch bei Zugabe von wässrigen Salzlösungen wie von Natriumsulfit, Natriumsulfat, Natriumphosphat oder Natriumcarbonat erfolgt und selbst bei Natriumsulfid, wenn dieses in zu geringer Menge zugesetzt wurde. Ggf. können auch andere Salze zugegeben werden. Um das Kupfer in ausreichendem Ausmaß zu entfernen, ist eine Zugabe von Natriumsulfid in mindestens äquiniolarer Menge, insbesondere in 1-bis 3-facher äquimolarer Menge, des vorhandenen Kupfersalzes erforderlich. Manchmal weist das Produkt nach der Fällung des Kupfersalzes als Kupfersulfid und dessen Abtrennung durch Filtrieren noch einen schwachen Geruch nach Schwefelwasserstoff auf. Ist dies der Fall, ist ein Desodorieren des Endproduktes möglich. Dies kann z.B. durch Hindurchleiten von Magerluft durch das auf etwa 60 bis 95 °C erwärmte Produkt bei einem Unterdruck von 10 bis 200 mbar erfolgen, bis kein Schwefelwasserstoffgeruch mehr festgestellt werden kann. Gleichzeitig wird das zusätzliche Wasser abdestilliert.

Das erfinderische Verfahren ist geeignet zur technischen Herstellung von Acrylatmonomeren, Reaktivverdünnern, Polyetheracrylaten und Polyesteracrylaten. Die erfindungsgemäß hergestellten Produkte sind insbesondere für Verwendungen geeignet, die helle Produkte benötigen, wie farbpigmentierte Beschichtungen auf Substraten wie Holz, Papier, Kunststoffe, mineralische Baustoffe, Metalle und beschichtete Metalle.

Die nachstehenden Beispiele und Vergleichsversuche sollen das erfinderische Verfahren weiter erläutern, aber nicht beschränken. Soweit nicht ausdrücklich anders angegeben, beziehen sich Teile und Prozente auf das Gewicht.

### Beispiel 1

622,2 g eines ethoxylierten Pentaerythrits, das durchschnittlich 5 Ethoxylgruppen pro Pentaerythrit-Molekül enthielt (PP50); 604,36 g Acrylsäure; 0,6 g Unterphosphorige Säure; 0,2 g Kupfer (II)-chlorid; 30,0 g Cyclohexan und 6,15 g Methansulfonsäure wurden gemischt und durch Erhitzen auf eine Reaktoraußentemperatur von 130 °C 4 Stunden unter Rühren und Durchperlen von Magerluft verestert, wobei 115 ml Wasser ausgekreist wurden. Aus dem resultierenden Ansatz mit einer Säurezahl von 62 mg KOH/g wurde nach der Entfernung des Cyclohexans die überschüssige Acrylsäure bei 130 °C bei einem Druck von 40 mbar unter Durchperlen von Magerluft abdestilliert. Nach einstündiger Destillation hatte der Ansatz eine Säurezahl von 10 mg KOH/g. Das so erhaltene säurehaltige PP50-Acrylat wurde nach dem Abkühlen auf 80 °C mit 20 g CaO (was 2 g CaO pro 100g PP50-Acrylat entspricht) neutralisiert. In verschiedenen Ansätzen wurde dann versucht, durch Zusätze verschiedener 25%iger wäßriger Salzlösungen das in einer Menge von je 0,02 % Kupfersalz im Ansatz enthaltende Kupfer (II) chlorid zu fällen. Die durchgeführten Versuche zeigt Tabelle 1.

**Tabelle 1**

| Versuche zur Fällung des Kupfer (II) chlorids | | | |
|---|---|---|---|
| Fällungsmittel | Menge (%) | Grünfärbung bei Zugabe von 6% Diethylamin | Vernetzung in 1 Woche bei Licht+ Raumtemp. |
| Na₂SO₃ | 0,02 | ja | ja |
| Na₂SO₄ | 0,02 | ja | ja |
| Na₃PO₄ | 0,02 | ja | ja |
| Na₂CO₃ | 0,02 | ja | ja |
| Seitz Ultra* | 0,02 | ja | ja |
| Sokalan CP5** | 0,02 | ja | ja |
| Na₂S | 0,01 | ja | ja |
| Na₂S | 0,015 | ja | ja |
| Na₂S | 0,02 | nein | nein |
| Na₂S | 0,03 | nein | nein |
| Na₂S | 0,04 | nein | nein |
| Na₂S | 0,06 | nein | nein |

| | | | |
|---|---|---|---|
| * Seitz Ultra: Schichtsilikat | | | |
| ** Sokalan CP5: Acrylsäure-Copolymer | | | |

Wie die Tab.1 zeigt, konnte nur mit Na₂S in einer Menge von mind. 0,02 % (bezogen auf den Acrylsäureester) eine ausreichende Fällung des Kupfersalzes erzielt werden. Diese Produkte waren als einzige bei Lagerung an Licht bei Raumtemperatur nach ca. 2 Wochen noch nicht vernetzt.

### Beispiel 2

Eine Mischung der in Beispiel 1 angegebenen Ausgangsprodukte wurde bei 130 °C Außentemperatur unter Rühren 4 Stunden verestert, wobei 118 ml Wasser ausgekreist wurden. Aus dem resultierenden Ansatz mit einer Säurezahl von 57 mg KOH/g wurde nach Entfernung des Cyclohexans die überschüssige Acrylsäure bei 130 °C Aussentemperatur und einem Druck von 40 mbar abdestilliert. Nach 1,5 stündiger Destillation wurde der säurehaltige Ansatz (Säurezahl 5,7 mg KOH/g) des PP50-Acrylats mit 11,4 g CaO neutralisiert. Durch Zugabe von 0,02 % Na₂S (als 25%-ige wäßrige Lösung) wurde das Kupfer als Kupfersulfid gefällt und vom PP50-Acrylat abfiltriert. Das filtrierte PP50-Acrylat wurde danach bei 95 °C und einem Druck von 60 bis 80 mbar durch Durchleiten von Magerluft desodoriert bis kein Schwefelwasserstoff mehr gerochen wurde. Das resultierende Produkt hatte eine Jodfarbzahl von unter 2 und einen Wassergehalt von 0,05%.

### Beispiel 3

Eine Mischung von 556,32 g ethoxyliertem Trimethylolpropan (TMP), das durchschnittlich 3 Ethoxygruppen pro Molekül Trimethylolpropan enthielt (TP30); 475,2 g Acrylsäure, 0,5 g Unterphosphorige Säure; 0,18 g Kupfer (II)-chlorid, 30,0 g Cyclohexan und 4,4 g Methansulfonsäure wurde während 5 Stunden bei 130 °C verestert, wobei 90 ml Wasser ausgekreist wurden. Nach Entfernung des Cyclohexans wurde bei 130 °C und einem Druck von 40 mbar überschüssige Acrylsäure während 1,5 Stunden abdestilliert. Das resultierende TP30-Acrylat mit einer Säurezahl von 5,7 mg KOH/g wurde auf 80 °C abgekühlt und mit 11,4 g CaO neutralisiert. Danach wurde durch Zugabe von 0,025% Natriumsulfid (als 25%-ige wäßrige Lösung) das Kupfer als Kupfersulfid gefällt und vom TP30-Acrylat abfiltriert. Das filtrierte TP30-Acrylat wurde dann wie in Beispiel 2 angegeben desodoriert bis kein Schwefelwasserstoff mehr gerochen werden konnte. Der resultierende Acrylester hatte eine Jodfarbzahl von 1-2 und einen Wassergehalt von unter 0,05 %.

### Beispiel 4

529,24 g propoxyliertes Trimethylolpropan (TMP), das durchschnittlich 3 Propoxylgruppen pro Molekül TMP enthielt (TS30), 432 g Acrylsäure, 0,48 g Unterphosphoriger Säure, 0,16 g Kupfer (II) chlorid, 30,0 g Cyclohexan und 4,0 g Methansulfonsäure wurden gemischt und bei einer Aussentemperatur von 130 °C unter Rühren 4 Stunden verestert, wobei 85 ml Reaktionswasser ausgekreist wurden. Nach Entfernung des Cyclohexan wurde während 1,5 Stunden bei 130 °C Aussentemperatur und bei einem Druck von 40 mbar Acrylsäure abdestilliert. Das erhaltene Reaktionsprodukt hatte eine Säurezahl von 6 mg KOH/g und wurde nach dem Abkühlen auf 80 °C mit 11,4 g Calciumoxyd neutralisiert. Durch Zugabe von 0,025 % Natriumsulfid (als 25%-ige wäßrige Lösung) wurde das Kupfersalz als Kupfersulfid gefällt und das TS30-Acrylat filtriert. Eine Desodorierung des Produktes, das eine Jodfarbzahl von 1-2 und einen Wassergehalt von unter 0,05% hatte, war nicht erforderlich.

### Beispiel 5

Eine Mischung von 472,0 g 1,6-Hexandiol, 691,2 g Acrylsäure, 0,18 g Kupfer (II) chlorid, 0,54 g Unterphosphorige Säure, 5,51 g Methansulfonsäure und 80,0 g Cyclohexan wurde durch Erhitzen auf eine Aussentemperatur von 130 °C (Innentemperatur 97-108 °C) unter Rühren verestert. Nach 4 Stunden waren 158 ml Wasser ausgekreist und die Veresterung praktisch abgeschlossen. Danach wurden das Cyclohexan und die überschüssige Acrylsäure bei 105 °C bei einem unter Einperlen von Magerluft eingestelltem Unterdruck von 60 bis 80 mbar während 1,5 Stunden abdestilliert. Das resultierende Zwischenprodukt hatte eine Säurezahl von 5,2 mg KOH/g und wurde mit 0,1% Methylethylhydrochinon stabilisiert. Durch Zusatz einer wässrigen Natriumsulfidlösung (0,27 g Natriumsulfid in 2 g Wasser) wurde das Kupfersalz als Kupfersulfid ausgefällt und das 1,6-Hexandiolacrylat mit 10%iger Natronlauge auf einen pH-Wert von 7-8 eingestellt. Das im Ansatz vorhandene Wasser wurde danach innerhalb von 1 Stunde bei 105 °C abdestilliert und das Produkt abfiltriert. Es resultierte ein helles Produkt mit einer Jodfarbzahl von kleiner als 2 und einem Wassergehalt von 0,05%.

### Beispiel 6

Es wurde wie in Beispiel 5 verfahren, jedoch wurde die Acrylsäure sowie das Wasser jeweils bei 95 °C abdestilliert. Die Produktqualität des Endproduktes entsprach der des gemäß Beispiel 5 hergestellten Produktes.

### Beispiel 7

Es wurde wie in Beispiel 5 verfahren, jedoch wurde als Veresterungskatalysator die 1,5 fache Menge an Methansulfonsäure verwendet. Die Säurezahl des Zwischenproduktes nach dem Abdestillieren der Acrylsäure betrug 8,2 mg KOH/g. Es wurde entsprechend mehr Natriumhydroxid für die Neutralisation verwendet.

### Beispiel 8

Es wurde wie in Beispiel 5 verfahren, jedoch zur Veresterung eine Mischung von 622,2 g eines ethoxylierten Pentaerythrits, das durchschnittlich 5 Ethoxylgruppen pro Molekül Pentaerythrit enthielt, 604,4 g Acrylsäure, 0,2 g Kupfer (II) chlorid, 0,6 g Unterphosphorige Säure, 6,2 g Methansulfonsäure und 80,0 g Cyclohexan verwendet. Die Veresterung und anschließende Aufarbeitung erfolgte entsprechend Beispiel 5. Das helle Endprodukt hatte eine Jodfarbzahl von 1-2 und einen Wassergehalt von unter 0,05 %.

## Patentansprüche

1. Verfahren zur Herstellung von (Meth) acrylsäureestern durch Veresterung von Acrylsäure und/oder Methacrylsäure mit mindestens einer Verbindung, die mindestens eine alkoholische Hydroxylgruppe aufweist, bei einer Temperatur von 90 bis 150 °C in Gegenwart eines sauren Veresterungskatalysators und eines Polymerisationsinhibitors unter Zugabe eines Kohlenwasserstoffes mit einem Siedepunkt im Bereich von 60 bis 140 °C als Wasserschleppmittel, wobei nach der Veresterung das Wasserschleppmittel und überschüssige (Meth) acrylsäure bei einer Temperatur von 90 bis 150 °C bei Normaldruck oder Unterdruck abdestilliert wird und gegebenenfalls das nach dem Abdestillieren erhaltene Produkt mit mindestens einer basischen anorganischen Verbindung neutralisiert wird, dadurch gekennzeichnet, daß als Polymerisationsinhibitor ein Kupfer (II) salz oder ein Gemisch aus einem Kupfer (II) salz und einem Kupfer (I) salz verwendet wird, das nach der Veresterung als Kupfersulfid gefällt und abgetrennt wird.

2. Verfahren nach Anspruch 1, wobei man als Katalysator Kupfer (II) chlorid verwendet.

3. Verfahren nach Anspruch 1 oder 2, wobei man den Katalysator durch Zugabe eines Alkalimetallsulfids oder -hydrogensulfids fällt.

4. Verfahren nach Anspruch 3, wobei man das Alkalimetallsulfid oder -hydrogensulfid in 1- bis 3-facher äquimolarer Menge zugibt.

5. Verfahren nach einem der vorhergehenden Ansprüche, dadurch gekennzeichnet, dass die zu veresternde Verbindung mit alkoholischen Hydroxylgruppen ein Oxalkylierungsprodukt eines aliphatischen mehrwertigen Alkohols darstellt.

6. Verfahren nach einem der vorhergehenden Ansprüche, dadurch gekennzeichnet, dass bei der Veresterung das entstehende Reaktionswasser entfernt wird.

7. Verfahren nach einem der vorhergehenden Ansprüche, dadurch gekennzeichnet, dass das nach dem Abdestillieren des Wasserschleppmittels und überschüssiger (Meth) acrylsäure erhaltene saure Produkt mit mindestens einer im (Meth) acrylester nicht löslichen basischen anorganischen Verbindung neutralisiert wird.

8. Verfahren nach einem der vorhergehenden Ansprüche, dadurch gekennzeichnet, dass das nach dem Abdestillieren des Wasserschleppmittels und überschüssiger (Meth) acrylsäure erhaltene saure Produkt mit Oxiden, Hydroxiden und/oder Carbonaten eines Alkalimetalls, Erdalkalimetalls und/oder des Aluminiums trocken neutralisiert wird.

9. Verfahren nach einem der vorhergehenden Ansprüche, dadurch gekennzeichnet, dass man während der Veresterung und/oder des Abdestillierens überschüssiger (Meth) acrylsäure ein Inertgas-Luft-Gemisch mit einem Sauerstoffgehalt von 1. bis 20 Volumenprozent durch das Reaktionsgemisch und/oder oberhalb von diesem durch den Reaktor leitet.

10. Verfahren nach einem der vorhergehenden Ansprüche, dadurch gekennzeichnet, dass die Fällung des Kupfersulfids nach vollständiger oder teilweiser Neutralisation des nach dem Abdestillieren überschüssiger (Meth) acrylsäure erhaltenen Produktes erfolgt.
